# EUROPÄISCHE PATENTSCHRIFT

(11) **EP 0 629 221 B1**
(45) Veröffentlichungstag und Bekanntmachung des Hinweises auf die Patenterteilung: **15.04.1998**
(21) Anmeldenummer: 93904005.1
(22) Anmeldetag: 22.02.1993
(51) Int. Cl.: C09B 51/00, A61K 7/13, C07D 237/28, D06P 3/70

(54) **FÄRBEMITTEL FÜR NATÜRLICHE UND SYNTHETISCHE FASERN**
COLOURING AGENTS FOR NATURAL AND SYNTHETIC FIBRES
COLORANTS POUR FIBRES NATURELLES ET SYNTHETIQUES

(30) Priorität: 02.03.1992 DE 4206537
(43) Veröffentlichungstag der Anmeldung: 21.12.1994
(73) Patentinhaber: Henkel Kommanditgesellschaft auf Aktien, 40191 Düsseldorf (DE)
(72) Erfinder: ROSE, David, D-4010 Hilden (DE); HÖFFKES, Horst, D-4000 Düsseldorf (DE); LIESKE, Edgar, D-4000 Düsseldorf (DE); MATZIK, Iduna, D-4020 Mettmann (DE)
(86) Internationale Anmeldenummer: EP9300414
(87) Internationale Veröffentlichungsnummer: WO9318093

(56) Entgegenhaltungen:
- EP-A- 0 353 531
- DE-A- 1 804 066
- GB-A- 519 695
- Voigt, Bornschein "Lehrbuch der pharmazeutischen Technologie" 5. Aufl.; Seite 475

## Beschreibung

Gegenstand der Erfindung sind neue Färbemittel für Fasern natürlichen Ursprungs und synthetische Fasern auf der Basis von N-substituierten 1,2,3,4-Tetrahydronitrochinoxalinen.

In der Färberei spielen die direktziehenden Farbstoffe eine herausragende Rolle.
In der Textilfärberei ist ein direktziehender Farbstoff definiert als "anionischer Farbstoff, der aus einem elektrolythaltigen wäßrigen Bad auf Cellulose aufzieht" (lt. "Committee of the Society of Dyers and Colourists" in "The Dyeing of Synthetic Polymer and Acetat Fibers", Dyers Company Publications Trust, 1979).

Im Sinne der vorliegenden Erfindung ist ein direktziehender Farbstoff jedoch ein Farbstoff, der ohne vorhergehende Behandlung der Faser aus einem für die jeweils zu erzielende Färbung geeigneten Medium auf die Faser aufzieht. Im Sinne der Erfindung ist der Begriff des direktziehenden Farbstoffs weiter gefaßt, was der vorliegenden Erfindung insofern Rechnung trägt, als die Verwendung der neuen Färbemittel nicht auf eine Faserart beschränkt ist.

Chinoxalinabkömmlinge im weitesten Sinne, wie zum Beispiel das Mauvein, sind in der Textilfärberei von großer Bedeutung. In diesen Verbindungen sind die beiden Ringstickstoffatome Teil des chromophoren Systems; sie unterscheiden sich damit grundlegend von den 1,2,3,4-Tetrahydronitrochinoxalinen, für deren Farbigkeit in erster Line der Nitrosubstituent verantwortlich ist und die deshalb der Klasse der Nitrofarbstoffe zuzuordnen sind. 1,2,3,4-Tetrahydronitrochinoxaline sind in der Textilfärberei unbekannt.

Aus der deutschen Offenlegungsschrift DE-A-38 25 212 sind 1,2,3,4-Tetrahydronitrochinoxaline und deren Salze als direktziehende Haarfarbstoffe bekannt. Sie bilden im allgemeinen zwar intensive Färbungen von guter Lichtechtheit, haben jedoch den Nachteil, daß sie in polaren Solventien wie Wasser oder Ethanol nur schwer löslich sind. Dies führt häufig zu ungleichmäßigen Färbungen, insbesondere bei Färbemitteln, die zur Erzielung bestimmter Nuancen hohe Konzentrationen an Farbstoffen enthalten. Aber auch bei Zubereitungen mit nur wenig solubilisierenden Trägerkomponenten besteht die Gefahr, daß schwächere Färbungen als vorgesehen erhalten werden, weil die Farbstoffe auskristallisieren und im Färbebad verbleiben, anstatt auf die Faser aufzuziehen.

Es wurde überraschenderweise gefunden, daß eine Reihe von neuen 1,2,3,4-Tetrahydrochinoxalinen diese genannten Nachteile nicht besitzen und daher die an direktziehende Farbstoffe zu stellenden Anforderungen in besonderem Maße erfüllen.

Gegenstand der Erfindung sind neue 1,2,3,4-Tetrahydrochinoxaline der allgemeinen Formel I 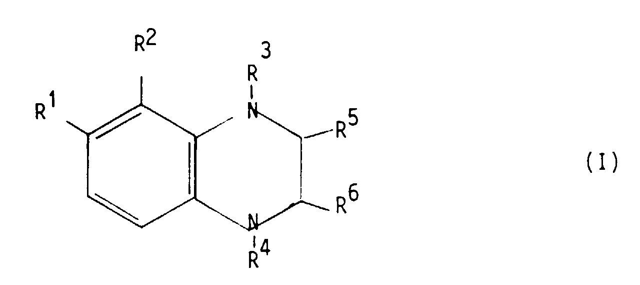 wobei eine der Gruppen R¹ und R² eine Nitrogruppe ist, während die zweite Wasserstoff, eine Alkylgruppe mit 1 bis 4 C-Atomen oder eine Alkoxygrppe mit 1 bis 4 C-Atomen ist, R³ und R⁴ unabhängig voneinander eine Gruppe -(CH₂-CHR⁷-O)ₙH darstellen, wobei R⁷ Wasserstoff, Methyl oder Ethyl bedeutet und n = 0 oder eine ganze Zahl von 1 bis 4 ist, mit der Maßgabe, daß in mindestens einer der Gruppen R³ und R⁴ n von 0 verschieden ist und R⁵ und R⁶ unabhängig voneinander Wasserstoff oder eine Alkylgruppe mit 1 bis 4 C-Atomen darstellen, und deren Salze.

Ein bevorzugter Gegenstand der Erfindung sind diejenigen Verbindungen der Formel I und deren Salze, in denen R¹ eine Nitrogruppe ist und in den Gruppen R³ und R⁴ n gleich 0 oder 1 ist und R⁷ Wasserstoff oder vorzugsweise Methyl bedeutet, mit der Maßgabe, daß in mindestens einer der Gruppen R³ und R⁴ n von 0 verschieden ist.

Ein weiterer Erfindungsgegenstand ist die Verwendung der 1,2,3,4-Tetrahydrochinoxaline der allgemeinen Formel I zum Färben von Fasern natürlichen Ursprungs und synthetischen Fasern.

Erfindungsgemäß sind unter Fasern natürlichen Ursprungs menschliche, tierische und pflanzliche Fasern wie Seide, Baumwolle, Leinen, Jute und Sisal, und Keratinfasern wie Haare, Pelze, Wolle oder Federn, aber auch regenerierte oder modifizierte Naturfasern, wie z. B. Viskose,Nitro- und Acetylcellulose, Alkyl-, Hydroxyalkyl- und Carboxyalkylcellulosen, zu verstehen. Aus der Klasse der synthetischen Fasern sind z. B. Polyamid-, Polyester- Polyacrylnitril- und Polyurethan-Fasern zu nennen.

Besonders gut zum Färben geeignet sind die 1,2,3,4-Tetrahydrochinoxaline der Formel I, bei denen R¹ eine Nitrogruppe ist und in den Gruppen R³ und R⁴ n gleich 0 oder 1 ist und R⁷ Wasserstoff oder vorzugsweise Methyl bedeutet, mit der Maßgabe, daß in mindestens einer der Gruppen R³ und R⁴ n von 0 verschieden ist.
Die besten Färbungen werden dabei erzielt, wenn R², R⁵ und R⁶ Wasserstoffatome sind.

Die Farbstoffe können sowohl selbst als auch in Form ihrer wasserlöslichen Salze eingesetzt werden. Salze werden in diesem Zusammenhang dann als wasserlöslich angesehen, wenn sie sich in einer zum Zwecke der Färberei ausreichenden Menge in Wasser oder wäßrigen Zubereitungen lösen.
Unter den wasserlöslichen Salzen sind in erster Linie die Hydrochloride, Hydrobromide u. ä. zu verstehen.
Es ist nicht erforderlich, daß eine einheitliche Verbindung der Formel I verwendet wird, vielmehr kann auch eine Mischung verschiedener Verbindungen der Formel I zum Einsatz kommen.

Die Farbstoffe der Formel I erzeugen Färbungen im Bereich von braunorange bis lackrot. Die Färbungen zeichnen sich durch sehr gute Licht-, Schweiß- und Waschechtheit aus.
Besonders überraschend sind die sehr guten Waschechtheiten der Färbungen hinsichtlich der Tatsache, daß die Farbstoffe der Formel I gut wasserlöslich sind. Die gute Wasserlöslichkeit der Farbstoffe ermöglicht ein rasches Aufziehen auf die zu färbende Faser aus wäßriger, bevorzugt alkalischer, Lösung.

Besonders gute Färbeergebnisse werden an Polyacrylnitrilfasern und keratinischen Fasern, vorzugsweise menschlichen Haaren, erzielt. Die Verwendung der 1,2,3,4-Tetrahydrochinoxaline der Formel I zum Färben dieser Fasern ist deshalb ein weiterer Erfindungsgegenstand.

Das Färben von Textilfasern geschieht vorzugsweise nach dem Ausziehverfahren bei Temperaturen über 90 °C, andere für das Färben von Textilfasern übliche Färbeverfahren sind jedoch ebenfalls geeignet.

Bevorzugt werden die 1,2,3,4-Tetrahydrochinoxaline jedoch zum Färben von menschlichen Haaren verwendet, da sie bereits bei physiologisch zuträglichen Temperaturen unterhalb 40 °C auf menschliches Haar aufziehen und dieses intensiv einfärben. Ein weiterer Erfindungsgegenstand sind deshalb Haarfärbemittel, die direktziehende Farbstoffe der Formel I oder deren Salze in einer Menge von 0,01 bis 5 Gew.-%, vorzugsweise 0,1 bis 2 Gew.-%, bezogen auf das gesamte Haarfärbemittel, und einen wasserhaltigen Träger enthalten.

Es ist zu beachten, daß in der Haarfärberei an die Farbstoffe und an die erzielten Färbungen besondere Anforderungen gestellt werden. Die Farbstoffe müssen dermatologisch und toxikologisch unbedenklich sein und bei niedrigen Temperaturen auf die Haare aufziehen. Die Färbungen müssen gegen Haarbehandlungsmethoden, wie z. B. Dauerwellen, beständig sein. Diesen hohen Anforderungen werden die 1,2,3,4-Tetrahydrochinoxaline der Formel I und die mit ihnen erzielten Färbungen in besonderem Maße gerecht.

Zum Färben von menschlichen Haaren werden die 1,2,3,4-Tetrahydrochinoxaline der Formel I bevorzugt in einen kosmetischen Träger, zum Beispiel Cremes, Emulsionen, Gele oder auch tensidhaltige schäumende Lösungen z. B. in Shampoos, Schaumaerosole oder andere Zubereitungen, die für die Anwendungen auf dem Haar geeignet sind, eingearbeitet.

Übliche Bestandteile solcher kosmetischer Zubereitungen sind z. B. Netz- und Emulgiermittel, wie anionische, nichtionische und ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäure und Alkylphenole, Sorbitanfettsäureester und Fettsäurepartialglyceride, Fettsäurealkanolaminde sowie Verdickungsmittel, wie z. B. Methyl- oder Hydroxycellulose, Stärke, Fettalkohole, Paraffinöle, Fettsäuren, ferner Parfumöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 bis 30 Gew.-% und Verdikkungsmittel in Konzentrationen von 0,1 bis 25 Gew.-% des gesamten Färbemittels eingesetzt.

Mit 1,2,3,4-Tetrahydrochinoxalinen der Formel I erzielt man brillante Färbungen im Rotbereich; Rotnuancen sind mit üblichen direktziehenden Farbstoffen nur schwer zu erreichen.

Zur Modifikation der Färbungen können den 1,2,3,4-Tetrahydrochinoxalinen der Formel I aber auch weitere übliche direktziehende Farbstoffe, wie z. B. Nitrobenzolderivate, Antrachinonfarbstoffe, Triphenylmethan oder Azofarbstoffe oder aber auch übliche Oxidationshaarfarbstoffvorprodukte, zugemischt werden. Bei den Oxidationshaarfarbstoffvorprodukten unterscheidet man Entwicklerkomponenten und Kupplerkomponenten. Entwicklerkomponenten bilden durch oxidative Kupplung untereinander oder gegebenenfalls in Gegenwart geeigneter Kupplerkomponenten die Oxidationshaarfarbstoffe aus. Als Entwicklersubstanzen werden z. B. primäre aromatische Amine mit einer weiteren in Para- oder Orthoposition befindlichen freien oder substituierten Hydroxy- oder Aminogruppe, Diaminopyridinderivate, heterocyclische Hydrazone, 4-Aninopyrrazolonderivate und 2,4,5,6-Tetraaminopyrrimidin und dessen Derivate eingesetzt. Als Kupplersubstanzen werden Metaphenylendiaminderivate, Naphthole, Resorcin und Resorcinderivate, Pyrrazolone und Metaaminophenole verwendet.

Weitere direktziehende Farbstoffe und Oxidationshaarfarbstoffvorprodukte können in einer Menge von 0,01 bis 5 Gew.-%, vorzugsweise von 1 bis 3 Gew.-%, enthalten sein.

Die Anwendung der erfindungsgemäßen Haarfärbemittel kann unabhängig von der Art der kosmetischen Zubereitung z. B. als Creme, Gel oder Shampoo im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

### Beispiele

### Herstellungsbeispiele

1. 1,2,3,4-Tetrahydro-N,N-bis-(β-hydroxyethyl)-6-nitrochinoxalinmonohydrochlorid (Farbstoff 1)
   Eine Mischung bestehend aus 44,75 g (0,25 Mol) 1,2,3,4-Tetrahydro-6-nitrochinoxalin, 75 ml Wasser und 75 ml Ethanol wurde im Autoklaven in Gegenwart von 33 g (0,75 Mol) Ethylenoxid auf 90 °C erhitzt. Nach 4 Stunden wurde abgekühlt und das Lösungsmittel im Vakuum entfernt. Das rote Öl wurde in 250 ml Aceton gelöst und mit einem Aceton/konz. HCl-Gemisch (10 : 1) gefällt. Der Niederschlag wurde abgesaugt und 2 mal aus je 100 ml halbkonzentrierter HCl (18 %ig) umkristallisiert. Das Produkt wurde anschließend bei 60 °C im Vakuum getrocknet.
   Ausbeute: 16,5 g, Schmelzpunkt: 140 - 152 °C
2. 1,2,3,4-Tetrahydro-N⁴-(β-hydroxyethyl)-6-nitrochinoxalinmonohydrochlorid (Farbstoff 2)
   Die Reaktionsführung und die Aufarbeitung erfolgten wie in Beispiel 1, aber mit nur 13,5 g (0,3 Mol) Ethylenoxid.
   Ausbeute: 22 g, Schmelzpunkte: 148 bis 168 °C
3. 1,2,3,4-Tetrahydro-N,N-bis-(β-hydroxyethyl)-6-nitrochinoxalinmonohydrochlorid (Farbstoff 1) und 1,2,3,4-Tetrahydro-N⁴-(β-hydroxyethyl)-6-nitrochinoxalinmonohydrochlorid (Farbstoff 2)
   Es wurde wie in Beispiel 1 verfahren.
   Der Niederschlag wurde aber nicht mehr umkristallisiert. DC-Untersuchungen zeigten, daß im nicht umkristallisierten Produkt eine Mischung aus mono- und diethoxylierten Produkten vorliegt.
   Ausbeute: 52,6 g
4. 1,2,3,4-Tetrahydro-N4-(β-hydroxypropyl)-6-nitrochinoxalinmonohydrochlorid (Farbstoff 3)
   Die Reaktionsführung und die Aufarbeitung erfolgten wie in Beispiel 1, aber mit 18 g (0,3 Mol) Propylenoxid.

### Anwendungsbeispiele

### Färben von Textilfasern:

Zum Färben der Textilfasern wurde ein Mehrfaserbegleitgewebe, Typ DW (entsprechend Deutsche Echtheitskommission) vom Zuschnitt 10 cm x 4 cm der Beuth Verlags GmbH, Köln verwendet. Ein solches Gewebe besteht aus 6 Textilstreifen, bestehend aus Acetylcellulose (2,5 Acetylgruppen pro Monomereinheit, Baumwolle), Polyamid, Polyester, Polyacryl und Wolle.

0,5 g des Farbstoffs, 8 g Natriumsulfat-decahydrat und 2 g Natriumcarbonat wurden bei 60 °C in 100 ml Wasser gelöst. Dem Färbebad wurde das Mehrfaserbegleitgewebe zugegeben. Anschließend wurde innerhalb von 45 Minuten auf eine Temperatur von 98 °C erhöht. Diese Temperatur wurde 1 Stunde lang beibehalten, wobei das verdampfte Wasser kontinuierlich ersetzt wurde. Danach wurde das gefärbte Gewebe zunächst mit kaltem und anschließend mit heißem Wasser gespült. Das Gewebe wurde anschließend 20 Minuten lang in 250 ml 0,25 g Natriumlaurylsulfat enthaltendem Wasser gekocht und anschließend gespült. Zum Schluß wurde das Gewebe getrocknet. Die Färbeergebnisse sind Tabelle 1 zu entnehmen.

**Tabelle 1**

| Fasertyp | Farbstoff 1 | Farbstoff 1 und 2 | Farbstoff 2 | Farbstoff 3 |
|---|---|---|---|---|
| Acetyl cellulose | nicht gefärbt | nicht gefärbt | nicht gefärbt | braunorange |
| | | | | |
| Baumwolle | nicht gefärbt | nicht gefärbt | nicht gefärbt | nicht gefärbt |
| | | | | |
| Polyamid | hellorange | grauorange | grauorange | braunorange |
| | | | | |
| Polyester | hellorange | orange | orange | orange |
| | | | | |
| Polyacryl | rotorange | rotorange | rotorange | rotorange |
| | | | | |
| Wolle | braunorange | braunorange | braunorange | hellbraun |

### Färben von menschlichen Haaren:

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄ + 2 E0 Sulfat, Na-Salz (28 %ig) | 25 g |
| Wasser | 60 g |
| direktziehender Farbstoff 1 bis 3 | 5,0 mMol |
| Ammoniumsulfat | 1 g |
| konz. Ammoniaklösung | bis pH = 9,5 |
| Wasser | ad 100 g |

Die Bestandteile wurden der Reihe nach miteinander vermischt. Nach Zugabe der direktziehenden Farbstoffe wurde mit konz. Ammoniaklösung der pH-Wert der Emulsion auf 9,5 eingestellt, dann wurde mit Wasser auf 100 g aufgefüllt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Das Ergebnis der Färbeversuche ist Tabelle 2 zu entnehmen.

**Tabelle 2**

| Farbstoff | Nuance des gefärbten Haares |
|---|---|
| 1 | rotbraun |
| 1 und 2 | lackrot |
| 2 | englischrot |
| 3 | rotorange |

## Patentansprüche

1. 1,2,3,4-Tetrahydrochinoxaline der allgemeinen Formel I 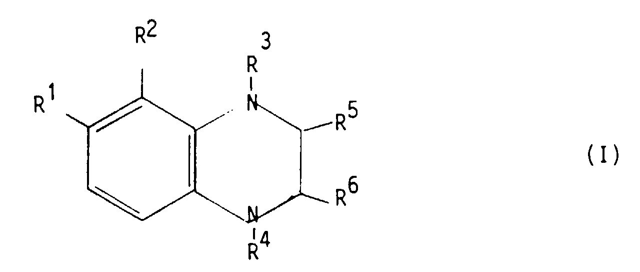 wobei eine der Gruppen R¹ und R² eine Nitrogruppe ist, während die zweite Wasserstoff, eine Alkylgruppe mit 1 bis 4 C-Atomen oder eine Alkoxygruppe mit 1 bis 4 C-Atomen ist, R³ und R⁴ unabhängig voneinander eine Gruppe -(CH₂-CHR⁷-O)ₙH darstellen, wobei R⁷ Wasserstoff, Methyl oder Ethyl bedeutet und n = 0 oder eine ganze Zahl von 1 bis 4 ist, mit der Maßgabe, daß in mindestens einer der Gruppen R³ und R⁴ n von 0 verschieden ist und R⁵ und R⁶ unabhängig voneinander Wasserstoff oder eine Alkylgruppe mit 1 bis 4 C-Atomen darstellen und deren Salze.

2. 1,2,3,4-Tetrahydrochinoxaline der Formel I und deren Salze nach Anspruch 1, dadurch gekennzeichnet, daß R¹ eine Nitrogruppe ist und daß in den Gruppen R³ und R⁴ n gleich 0 oder 1 ist und R⁷ Wasserstoff oder vorzugsweise Methyl bedeutet, mit der Maßgabe, daß in mindestens einer der Gruppen R³ und R⁴ n von 0 verschieden ist.

3. Verwendung von 1,2,3,4-Tetrahydrochinoxalinen der Formel I oder deren Salzen zum Färben von Fasern natürlichen Ursprungs und synthetischen Fasern.

4. Verwendung von 1,2,3,4-Tetrahydrochinoxaline der Formel I oder deren Salze nach Anspruch 3, dadurch gekennzeichnet, daß R¹ eine Nitrogruppe ist und daß in den Gruppen R³ und R⁴ gleich 0 oder 1 ist und R⁷ Wasserstoff oder vorzugsweise Methyl bedeutet, mit der Maßgabe, daß in mindestens einer der Gruppen R³ und R⁴ n von 0 verschieden ist.

5. Verwendung von 1,2,3,4-Tetrahydrochinoxalinen der Formel I oder deren Salzen zum Färben von Polyacrylnitrilfasern und keratinischen Fasern, vorzugsweise menschlichen Haaren.

6. Haarfärbemittel, die Farbstoffe der Formel I oder deren Salze in einer Menge von 0,01 bis 5 Gew.-%, vorzugsweise 0,1 bis 2 Gew.-%, bezogen auf das gesamte Haarfärbemittel, und einen wasserhaltigen Träger enthalten.

## Claims

1. 1,2,3,4-Tetrahydroquinoxalines corresponding to general formula I: 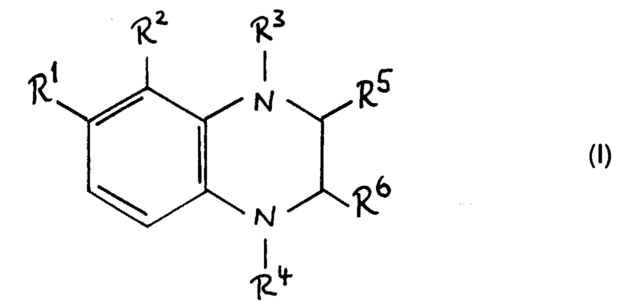 in which one of the substituents R¹ and R² is a nitro group while the other is hydrogen, an alkyl group containing 1 to 4 carbon atoms or an alkoxy group containing 1 to 4 carbon atoms, R³ and R⁴ independently of one another represent a -(CH₂-CHR⁷-O)ₙH group where R⁷ is hydrogen, methyl or ethyl and n = 0 or an integer of 1 to 4, with the proviso that, in at least one of the substituents R³ and R⁴, n is not 0, and R⁵ and R⁶ independently of one another represent hydrogen or an alkyl group containing 1 to 4 carbon atoms, and salts thereof.

2. 1,2,3,4-Tetrahydroquinoxalines corresponding to formula I and salts thereof as claimed in claim 1, characterized in that R¹ is a nitro group and, in the substituents R³ and R⁴, n = 0 or 1 and R⁷ represents hydrogen or preferably methyl, with the proviso that, in at least one of the substituents R³ and R⁴, n is not 0.

3. The use of 1,2,3,4-tetrahydroquinoxalines corresponding to formula I or salts thereof for colouring fibres of natural origin or synthetic fibres.

4. The use of 1,2,3,4-tetrahydroquinoxalines corresponding to formula I or salts thereof as claimed in claim 3, characterized in that R¹ is a nitro group and, in the substituents R³ and R⁴, n = 0 or 1 and R⁷ represents hydrogen or preferably methyl, with the proviso that, in at least one of the substituents R³ and R⁴, n is not 0.

5. The use of 1,2,3,4-tetrahydroquinoxalines corresponding to formula I or salts thereof for colouring polyacrylonitrile fibres and keratin fibres, preferably human hair.

6. Hair colorants containing dyes corresponding to formula I or salts thereof in a quantity of 0.01 to 5% by weight and preferably in a quantity of 0.1 to 2% by weight, based on the hair colorant as a whole, and a water-containing carrier.

## Revendications

1. 1,2,3,4-Tétrahydroquinoxalines de formule I 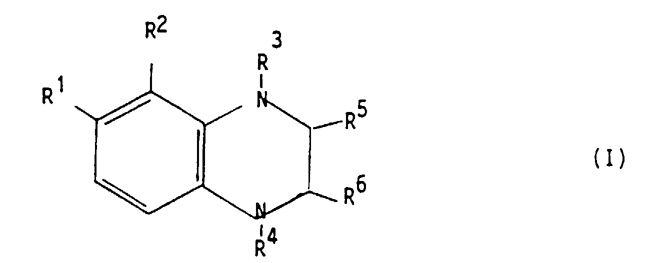 dans laquelle l'un des groupes R¹ et R² est un groupe nitro, tandis que le second est un hydrogène, un groupe alkyle ayant de 1 à 4 atomes de carbone ou un groupe alcoxy ayant de 1 à 4 atomes de carbone, R³ et R⁴ représentent indépendamment l'un de l'autre un groupe -(CH₂-CHR⁷O)ₙH, où R⁷ représente un hydrogène, un méthyle ou un éthyle et n = O ou un nombre entier compris entre 1 et 4, sous réserve que dans au moins l'un des groupes R³ et R⁴ n est différent de O et R⁵ et R⁶ représentent indépendamment l'un de l'autre un hydrogène ou un groupe alkyle ayant de 1 à 4 atomes de carbone, et leurs sels.

2. 1,2,3,4 -Tétrahydroquinoxalines de formule I et leurs sels selon la revendication 1,
caractérisées en ce que
• R¹ est un groupe nitro, et
• dans les groupes R³ et R⁴ n est égal à O ou 1 et R⁷ représente un hydrogène ou de préférence un méthyle, sous réserve que dans au moins l'un des groupes R³ et R⁴ n est différent de 0.

3. Utilisation des 1,2,3,4-tétrahydroquinoxalines de formule I ou de leurs sels pour la coloration de fibres d'origine naturelle et synthétiques.

4. Utilisation de 1,2,3,4-tétrahydroquinoxalines de formule I ou de leurs sels selon la revendication 3,
caractérisée en ce que
• R¹ est un groupe nitro, et
• dans les groupes R³ et R⁴ n est égal à O ou 1 et R⁷ représente un hydrogène ou de préférence un méthyle, sous réserve que dans au moins l'un des groupes R³ ou R⁴ n est différent de 0.

5. Utilisation de 1,2,3,4-tétrahydroquinoxalines de formule I ou de leurs sels pour la coloration de fibres de polyacrylonitrile et de fibres kératiniques, de préférence de cheveux humains.

6. Agents colorants pour cheveux, qui contiennent des colorants de formule I ou leurs sels en une quantité de 0,01 à 5 % en poids, de préférence de 0,1 à 2 % en poids, par rapport à l'ensemble de l'agent colorant, et un support contenant de l'eau.
